Europäisches Patentamt

European Patent Office

Office européen des brevets

(19)

(11) Veröffentlichungsnummer: **0 423 062 A2**

# (12) EUROPÄISCHE PATENTANMELDUNG

(21) Anmeldenummer: **90730012.3**

(22) Anmeldetag: **04.10.90**

(51) Int. Cl.⁵: **A61B 1/12, A61B 1/00**

(30) Priorität: **07.10.89 DE 3933856**

(43) Veröffentlichungstag der Anmeldung:
**17.04.91 Patentblatt 91/16**

(84) Benannte Vertragsstaaten:
**AT BE CH DE DK ES FR GB GR IT LI LU NL SE**

(71) Anmelder: **Wiest, Peter P., Dipl.-Ing.**
**Hessenallee 8**
**W-1000 Berlin 19(DE)**

(72) Erfinder: **Wiest, Peter P., Dipl.-Ing.**
**Hessenallee 8**
**W-1000 Berlin 19(DE)**

(74) Vertreter: **Lüke, Dierck-Wilm, Dipl.-Ing.**
**Gelfertstrasse 56**
**W-1000 Berlin 33(DE)**

(54) **Vorrichtung zum Spülen und Absaugen von Körperhöhlen.**

(57) Die Erfindung bezieht sich auf eine Vorrichtung zum Spülen und Absaugen von Körperhöhlen, insbesondere bei der operativen Laparoskopie, mit einer Druck- und einer Saugpumpe und mit an diese angeschlossenen Schlauchleitungen zur Körperhöhle.

Um eine Vorrichtung zum Spülen und Absaugen von Körperhöhlen zu schaffen, welche sehr einfach zu bedienen ist, den Sterilitätsanforderungen voll entspricht und bei welcher sich sowohl der Absaugdruck als auch der Spüldruck auf einfache Weise fein steuern lassen, sieht die Erfindung vor, daß eine Steuervorrichtung 1 zum wechselweisen Spülen und Absaugen der Körperhöhle vorgesehen ist, daß die Steuervorrichtung 1 an eine Steuerleitung 3 und ein in dieser angeordnetes Steuerventil 20 angeschlossen ist und in Abhängigkeit von dieser die Druck- und Saugpumpe 13,14 steuert und daß die Einstellung des Gasballastes in der Steuerleitung 3 mittels des Steuerventiles 20 eine Steuerung des Druckes und /oder der Drehzahl der Druck- und Saugpumpe 13,14 bewirkt.

FIG.2

EP 0 423 062 A2

## VORRICHTUNG ZUM SPÜLEN UND ABSAUGEN VON KÖRPERHÖHLEN

Die Erfindung bezieht sich auf eine Vorrichtung zum Spülen und Absaugen von Körperhöhlen, insbesondere bei der operativen Laparoskopie, mit einer Druck- und einer Saugpumpe und mit an diese angeschlossenen Schlauchleitungen zur Körperhöhle.

Bei einer bekannten Vorrichtung dieser Art wird die Spülwasserzufuhr über eine Einmal-Spülwasserflasche aus Glas durchgeführt, bei welcher mittels eines tief nach unten eingestochenen Ausflußdornes und mittels von oben eingepreßter Luft der nötige Spüldruck erzeugt wird. Die Spülung wird durch ein Ventil an der Vorrichtung freigegeben. Für die Absaugung wird eine Auffangflasche verwendet, die von einer nur für Gase geeigneten Pumpe evakuiert wird. Die Absaugung wird ebenfalls an einem an der Vorrichtung angebrachten Ventil freigegeben.

Nachteilig hierbei ist die schlechte Steuerbarkeit der Absaugung allein durch die Stellung des Absaugventils an der Vorrichtung. Ein minimal geöffnetes Absaugventil kann durch angesaugte Blutkoagel schnell verstopfen. Zusätzlich ist häufig eine Leerung der Auffangflasche nötig. Wenn diese Leerung vergessen wird, erfolgt bei einem Verkleben der Überlaufsicherung ein Ansaugen von Sekret in die VakuumSaugpumpe, die dabei unbrauchbar wird.

Ferner ist die Handhabung der Spülflasche sehr umständlich und zeitraubend. Hierzu müssen ein dicker Dorn zur Flüssigkeitsentnahme und ein zweiter Dorn zur Luftauffüllung der Spülwasserflasche zwecks Druckerzeugung eingestochen werden. Außerdem ergeben sich hygienische Probleme sowohl durch Hereinpumpen von ungefilterter Luft in die Flasche als auch durch die schwierige Handhabung mit dem übergroßen Einstichdorn. Außerdem läßt sich der Spülstrahl in Form und Stärke wenig verändern. Schließlich ist der Anwender auf die Lieferform für die Spülflüssigkeitsflasche festgelegt.

Der Erfindung liegt von daher die Aufgabe zugrunde, eine Vorrichtung zum Spülen und Absaugen von Körperhöhlen der gattungsgemäßen Art zu schaffen, welche sehr einfach zu bedienen ist, den Sterilitätsanforderungen voll entspricht und bei welcher sich sowohl der Absaugdruck als auch der Spüldruck auf einfache Weise fein steuern lassen.

Die Lösung dieser Aufgabe ergibt sich aus den kennzeichnenden Merkmalen des Anspruches 1. Die erfindungsgemäße Vorrichtung zum Spülen und Absaugen von Körperhöhlen umfaßt eine Steuervorrichtung für die Druck- und die Saugpumpe sowie ein Steuerventil zur Steuerung des Druckes und/oder der Drehzahl der Druck-und der Saugpumpe. Hierdurch wird eine Feinsteuerung sowohl des Absaugdruckes als auch des Spüldruckes auf einfache Weise ermöglicht, so daß die erfindungsgemäße Vorrichtung sehr einfach zu bedienen ist.

In der bevorzugten Ausführungsform ist das Steuerventil im Bereich des Handgriffes eines medizinischen Instrumentes angeordnet und die an die Druck-und die Saugpumpe angeschlossenen Schlauchleitungen sind an zwei Anschlußstutzen des mit hohlem Schaft versehenen medizinischen Instrumentes anschließbar. Hierdurch hat der Operateur das Steuerventil regelmäßig in seinem Wirkungsbereich und kann die Druck- und die Saugpumpe vom Instrument her bedienen und damit sowohl die Spülung als auch die Absaugung exakt steuern. Der großen Sicherheit wegen ist die Steuervorrichtung mit einer Vakuumpumpe versehen, welche über eine Schlauchleitung durch das Steuerventil Luft ansaugt .

In weiterer bevorzugter Ausbildung der Erfindung ist das medizinische Instrument doppelläufig ausgebildet, wobei das im Durchmesser kleinere Spülrohr innerhalb des im Durchmesser größeren Saugrohres angeordnet ist. Das Steuerventil ist im Handgriff des medizinisches Instrumentes angeordnet und mittels einer Rasteinrichtung in verschiedene, unterschiedliche Düsenöffnungen für ein Nadelventil einstellbar. Am Auslaß des Spülrohres ist innerhalb des medizinischen Instrumentes ein weiteres Nadelventil angeordnet, dessen Ventilnadel zur Ausbildung unterschiedlicher Ventilöffnungen einstellbar ist.

In weiterer, besonders vorteilhafter Ausbildung der Erfindung ist die Druck- und die Saugpumpe aus zwei Peristaltik-/Rollenrad-Pumpen gebildet, die mittels eines Umschaltgetriebes abwechselnd zum Spülen und Saugen einstellbar und von einem Antriebsmotor gemeinsam antreibbar sind. Die beiden Pumpenrollenräder sind auf koaxial ineinander angeordneten Achsen des Umschaltgetriebes gelagert. Beide Peristaltikpumpen haben einen gemeinsamen Anpreßbügel. Somit dienen zum Spülen und Absaugen zwei Peristaltikpumpen, die wechselseitig zum Spülen und Saugen mittels des Umschaltgetriebes und eines gemeinsamen Motors angetrieben werden. Sehr vorteilhaft ist die axiale Anordnung beider Rollenräder zueinander.

Dies ermöglicht die Zusammenfassung der beiden Pumpenschläuche in einer gemeinsamen Halterung, welche formschlüssigmit der Pumpenkombination ausgebildet ist und ein falsches Einlegen vermeidet. Zusätzlich preßt ein Anpreßbügel beide Schläuche an die jeweiligen Rollenräder, wodurch die Handhabung sehr vereinfacht wird.

Die erfindungsgemäße Vorrichtung dient insbesondere zur Anwendung in der operativen Laparoskopie und hier zum Abspülen von blutkoagulumbedecktem Gewebe. Ferner dient die Vorrichtung zum Ausspülen bzw. Abscheiden von nebel-und dampfförmigen Rückständen in der abdominellen Gasblase während oder nach der Anwendung eines chirurgischen Lasers und zum Aufsaugen von Körperflüssigkeiten und dem Spülmedium.

Die Erfindung ist nachfolgend anhand eines in den Zeichnungen dargestellten Ausführungsbeisnieles näher erläutert. Es zeigen:

Fig. 1 eine Prinzipdarstellung der Vorrichtung,

Fig. 2 die schaltungstechnische Anordnung der Steuervorrichtung,

Fig. 3 das an die Vorrichtung angeschlossene medizinische Instrument mit Anschluß für die Steuervorrichtung,

Fig. 4 eine vergrößerte Darstellung des distalen Endes des medizinischen Instrumentes in einer abgewandelten Ausführungsform,

Fig. 5 eine vergrößerte Schnittdarstellung durch ein Schlauchset für zwei koaxial angeordnete Peristaltikpumpen und

Fig. 6 eine einfachere Ausführungsform des med.Instrumentes.

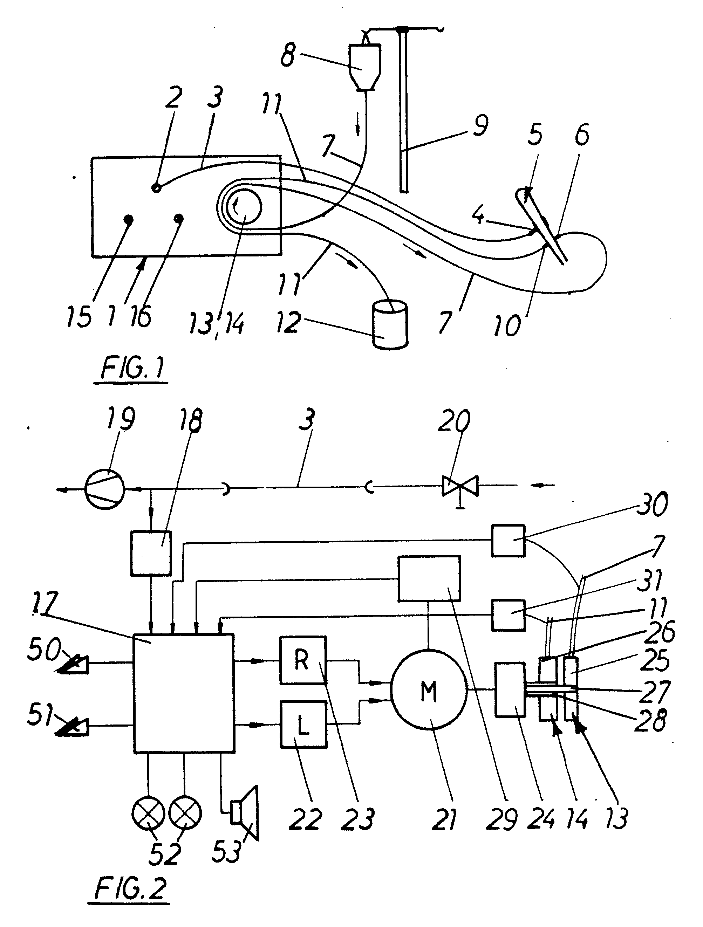

Die Figur 1 zeigt die in einem Gehäuse untergebrachte Steuervorrichtung 1 mit einem Anschluß 2 für eine Steuerleitung 3, die zu einem Anschlußstutzen 4 an einem medizinischen Instrument 5 geführt ist. An einen weiteren Anschlußstutzen 6 ist eine Spülleitung 7 angeschlossen, die zu einem Vorratsbehälter 8 für Spülflüssigkeit geführt ist, der an einem Ständer 9 aufgehängt ist. An einen weiteren Anschlußstutzen 10 des medizinischen Instrumentes 5 ist eine Saugleitung 11 angeschlossen, die zu einem Auffangbehälter 12 geführt ist. Die Spülleitung 7 und die Absaugleitung 11 sind um als Rollenrad-/Peristaltikpumpen 13, 14 ausgebildete Druck- und Saugpumpen herumgeführt, wie es später noch näher ausgeführt werden wird. Ein Netzschalter 15 und eine Kontrolleuchte 16 dienen zum Betrieb bzw. zur Überwachung der Vorrichtung.

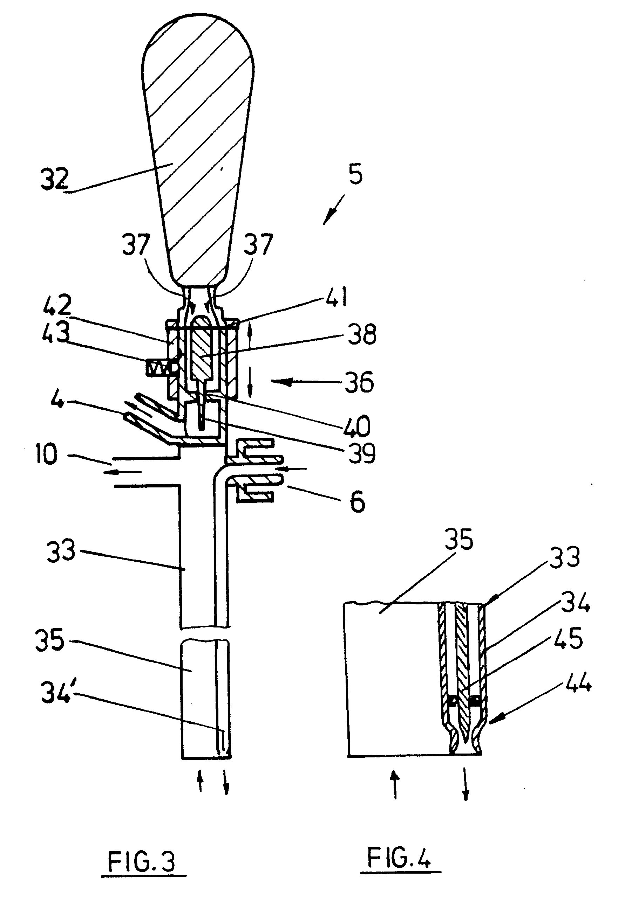

Die Figur 2 zeigt die im Gehäuse untergebrachte Steuervorrichtung 1. Diese umfaßt eine Auswerteelektronik 17, an welche die Steuerleitung 3 über einen Drucksensor 18 angeschlossen ist. In die Steuerleitung 3 sind eine Vakuumpumpe 19 und ein Steuerventil 20 eingesetzt, das Bestandteil des medizinischen Instrumentes 5 ist, wie es später anhand der Figur 3 noch näher erläutert werden wird. An die Auswertelektronik 17 ist ein Antriebsmotor 21 für die Peristaltikpumpen 13, 14 über zwei Motorleistungselemente 22, 23 angeschlossen, von denen das Element 22 für den Linkslauf und das Element 23 für den Rechtslauf des Elektromotors 21 dient. Dieser ist über ein Umschaltgetriebe 24 mit den beiden Peristaltikpumpen 13, 14 verbunden, deren Rollenräder 25, 26 auf koaxialen Achsen 27, 28 gelagert sind, die jeweils im Umschaltgetriebe 24 gelagert sind. Der Motor 21 ist ferner mit einem Istwertgeber 29 für die Drehzahl versehen, der ebenfalls mit der Auswerteelektronik 17 verbunden ist. Der Istwertgeber 29 erlaubt den Sollwert- Istwert- Vergleich der Motordrehzahl mittels der Auswerteelektronik 17 und damit den Sollwert-Istwert-Vergleich der Förderleistung beider Peristaltikpumpen 13, 14.

Die Auswerteelektronik 17 ist ferner mit Drucksensoren 30, 31 verbunden, die der Spülleitung 7 bzw.der Absaugleitung 11 zugeordnet sind. Die Drucksensoren 30,31 ermöglichen der Auswerteelektronik 17 eine Steuerung der Vorrichtung 1 nach gewünschtem Spül- und Saugdruck.

Die Figur 3 zeigt einen prinzipiellen Längsschnitt durch das medizinische Instrument 5. Dieses umfaßt einen Handgriff 32 an einem Ende und eine doppelläufige Kanülenadel 33 am anderen Ende. Hierbei ist das im Durchmesser kleinere Spülrohr 34 innerhalb des im Durchmesser größeren Saugrohres 35 angeordnet. Dementsprechend sind der Anschlußstutzen 6 an dem Spülrohr 34 und der Anschlußstutzen 10 an dem Saugrohr 35 angeschlossen. An den Anschlußstutzen 6 sinddie Spülleitung 7 und an den Anschlußstutzen 10 die Saugleitung 11 angeschlossen.

Zwischen den Anschlußstutzen 6, 10 und dem Handgriff 32 ist der Anschlußstutzen 4 für die Steuerleitung 3 ausgebildet. Der Anschlußstutzen 4 ist über ein Nadelventil 36 mit einer Ansaugöffnung 37 für Steuerluft versehen. Das Nadelventil 36 besteht aus einem Nadelschaft 38 und einer Steuernadel 39, die in eine Ventilöffnung 40 eingreift. Der Nadelschaft 38 ist über einen Stift 41 mit einer Außenhülse 42 verbunden, die mittels einer Rasteinrichtung 43 in eine Mittenstellung einstellbar und in beiden Richtungen stufenlos verstellbar ist. Die Pumpen 13,14 sind somit stufenlos steuerbar und in die neutrale Mittenstellung einstellbar .

Die Figur 4 zeigt eine weitere Ausführungsform der Kanülennadel 33 des medizinischen Instrumentes 5. Hierbei ist am Auslaß des Spülrohres 33 ein Nadelventil 44 angeordnet, dessen Ventilnadel 45 zur Ausbildung unterschiedlicher Ventilöffnungen einstellbar ist. Hierdurch wird die Spülströmung zu einem Sprühkegel verstellbar, mit dem die Gasfüllung im Abdomen von Nebel-und Dampfrückständen bei Anwendung eines chirurgischen Lasers ausgespült werden kann.

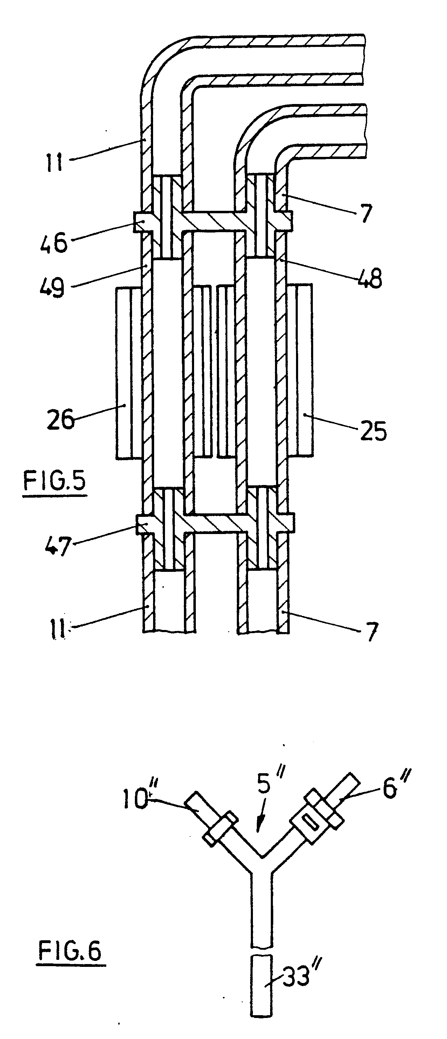

Die Figur 5 zeigt ein Schlauchset für die beiden mit koaxial ineinander angeordneten Achsen 27, 28 im Umschaltgetriebe 24 gelagerten Peristaltikpumpen 13, 14. Hierbei sind die Soülleitung 7 und die Saugleitung 11 unterteilt und durch gemeinsame Schlauchhalterungen 46, 47 miteinander verbunden. Die zwischen den Schlauchhalterungen 46 , 47 befindlichen Teile von Spülleitung 7 und Saugleitung 11 sind um die jeweiligen Rollenräder 25, 26 der Peristaltikpumpe 13,14 herumgelegt. Diese haben einen gemeinsamen Anpreßbügel.

Die erfindungsgemäße Vorrichtung arbeitet wie folgt: Nach Einschaltung der Steuervorrichtung 1 wird das am Nadelventil 36 des medizinischen Instrumentes 5 eingestellte Vakuum mittels des Drucksensors 18 gemessen. Die Auswerteelektronik 17 steuert in Abhängigkeit vom Vakuum-Eingangssignal den Antriebsmotor 21 entweder im Linkslauf 22 oder im Rechtslauf 23. Wenn das Drucksignal als Vakuum -proportionale Spannung aus dem mit einem Verstärker versehenen Drucksensor 18 im mittleren Bereich liegt, erfolgt keine Ansteuerung des Antriebsmotors 21 und damit erfolgen weder ein Spülen noch ein Absaugen. Je nach Drehrichtung des Antriebsmotors 21 wird das Rollenrad 25 zum Spülen oder das Rollenrad 26 zum Absaugen betrieben. Beim Betrieb des Rollenrades 25 zum Spülen führt die als Schlauch ausgebildete Spülleitung 7 vom Vorratsbehälter 8 Spülwasser zum Schlauch 48, der um das Rollenrad 25 der Peristaltikpumpe 13 herumgelegt ist. Von hier führt die Spülleitung 7 das Spülwasser zum Spülrohr 34 innerhalb des medizinischen Instrumentes 5. Wenn das Pumpenrad 26 der Peristaltikpumpe 14 zum Absaugen betrieben wird, wird die abzusaugende Flüssigkeit über das Rohr 35 des medizinischen Instrumentes der Saugleitung 11 zugeführt. Die ebenfalls als Schlauch ausgebildete Saugleitung 11 führt die abzusaugende Flüssigkeit dann dem um das Rollenrad 26 herumgelegten Pumpenschlauch 49 zu, von wo es über die Absaugleitung 11 dem Auffangbehälter 12 zugeführt wird. Der Vorratsbehälter 8 und der Auffangbehälter 12 können auch als Beutel ausgeführt sein. Der Auffangbehälter 12 kann so mit Inhalt entsorgt werden. Die beiden Schlauchhalterungen 46, 47 sind derart ausgebildet, daß diese wegen ihrer Formschlüssigkeit mit den beiden Peristaltikpumpen 13, 14 gar nicht anders plaziert werden können. Somit läßt sich das komplette Schlauchset sehr vorteilhaft als Einmalset ausbilden. Dadurch, daß dann weder ein Auffangbehälter noch eine Auffang flasche mit Überlaufsicherung ausgewaschen werden müssen, ist das Bedienungspersonal geringerer Infektionsgefahr ausgesetzt. Durch die im Gegensatz zur evakuierten Auffangflasche recht schnell änderbare Saugleistung der Rollenpumpe 14 lassen sich sehr kleine Flüssigkeitslachen absaugen, ohne daß sich das medizinische Instrument 5 am Gewebe festsaugt. Das Umschaltgetriebe 24 ermöglicht den kompakten und gewichtsparenden Aufbau der Vorrichtung.

Das medizinische Instrument 5, welches mittels eines Trokars in die Körperhöhle eingeführt wird, kann im einfachsten Fall ein dünnwandiges Rohr mit einem Außendurchmesser von ca. 5 mm sein. Zwischen dem proximalen Ende und dem Handgriff 32 des Instrumentes 5 sind die Anschlußstutzen 6, 10 für die Spülleitung 7 und die Absaugleitung 11 angeordnet. Dadurch, daß die als Rollenpumpen ausgebildeten Peristaltikpumpen 13,14 die Schlauchleitungen 48, 49 der Spülleitung 7 und der Absaugleitung 11 verschließen, ist kein Ventil an der Vorrichtung nötig. Die Kanülennadel 33 ist Spülrohr und Absaugrohr zugleich.

Die in Fig. 4 dargestellte Ausführung des distalen Endes des medizinischen Instrumentes 5 zeigt, daß das Spülrohr 34 als Innenrohr des Instrumentes 5 einen Düsenauslaß aufweist, der mit einem Nadelventil 44 versehen ist. Dies ermöglicht dem Operateur, je nach Leistung der zugeordneten Rollenpumpe 13 einen energiereicheren oder energiesanfteren Strahl zu erzeugen, ohne unnötig viel an Spülflüssigkeit verwenden zu müssen. Der Restquerschnitt der Kanülennadel 33 wird zum Absaugen als Absaugrohr 35 verwendet.

Wenn die Auslaßdüse des Nadelventils 44 zur am Instrument 5 verstellbaren Düse erweitert wird, mit welcher einerseits ein zusammenhängender Spülstrahl und andererseits ein in sich ziemlich homogener Sprühkegel von mindestens 90 ° erzeugt wird,so lassen sich einerseits Gewebe vom Koagulum freispülen und andererseits auch Nebel und Dämpfe bei Anwendung des chirurgischen Lasers aus der Gasblase im Abdomen auswaschen. Die Veränderung der axialen Position des Ventilkegels gegen das Spülrohr 34 macht diese Variation möglich. Physiologisch unbedenkliche Spülflüssigkeitszusätze können das Auswaschen der Laser-Rückstände noch steigern. Die Erzeugung eines homogenen Sprühkegels erfordert einen höheren Staudruck von ca. 1 bar vor der Auslaßdüse in Form des Nadelventils 44, der am einfachsten mit der Rollenpumpe 13 erzeugt wird.

Zur Steuerung des Saug-und des Spülvorganges besitzt das medizinische Instrument 5 den Anschlußstutzen 4, welcher zum Nadelventil 36 mündet, das mit der verschiebbaren Außenhülse 42 um eine Mittenstellung herum hin - und her positioniert werden kann. Zusätzliche Möglichkeiten zum Steuern der Peristaltik-Pumpenkombination mit Fußpedalen für Rechts- und Linkslauf des Motors 21 sind durch an die Steuervorrichtung 17 angeschlossenenFußpedale 50, 51 gegeben. Ferner sind der Steuervorrichtung 17 optische und akustische Signalerzeuger 52, 53 zugeordnet, welcher die Betriebszustände der Vorrichtung darstellen.

Die Figur 6 zeigt eine vereinfachte Ausführungsform des medizinischen Instrumentes 5", bei welchem die Anschlußstutzen 6" und 10" in eine gemeinsame Kanülenadel 33" münden .

**Ansprüche**

1. Vorrichtung zum Spülen und Absaugen von Körperhöhlen, insbesondere bei der operativen Laparoskopie,mit einer Druck- und einer Saugpumpe und mit an diese angeschlossenen Schlauchleitungen zur Körperhöhle, **dadurch gekennzeichnet,** daß eine Steuervorrichtung(1)zum wechselweisen Spülen und Absaugen der Körperhöhle vorgesehen ist,
daß die Steuervorrichtung(1) an eine Steuerleitung ( 3 ) und ein in dieser angeordnetes Steuerventil ( 20 ) angeschlossen ist und in Abhängigkeit von dieser die Druck-und Saugpumpe ( 13,14 ) steuert , und daß die Einstellung des Gasballastes in der Steuerleitung ( 3 ) mittels des Steuerventiles ( 20 ) eine Steuerung des Druckes und/ oder der Drehzahl der Druck-und Saugpumpe ( 13,14 ) bewirkt.

2. Vorrichtung nach Anspruch 1, dadurch gekennzeichnet, daß die Steuervorrichtung ( 1 ) eine an die Steuerleitung ( 3 ) angeschlossene Vakuumpumpe ( 19 ) umfaßt.

3. Vorrichtung nach Anspruch 1 oder 2, dadurch gekennzeichnet, daß das Steuerventil ( 20 ) im Bereich des Handgriffes ( 32 ) eines medizinischen Instrumentes ( 5 ) angeordnet ist und daß die an die Druck-und die Saugpumpe ( 13, 14 ) angeschlossenen Schlauchleitungen ( 7,11 ) an zwei Anschlußstutzen ( 6,10 ) des mit hohlem Schaftversehen medizinischen Instrumentes ( 5 ) anschließbar sind.

4. Vorrichtung nach Anspruch 3, dadurch gekennzeichnet, daß das medizinische Instrument ( 5 ) doppelläufig ausgebildet ist, wobei das im Durchmesser kleinere Spülrohr ( 34 ) innerhalb des im Durchmesser größeren Saugrohres ( 35 ) angeordnet ist.

5. Vorrichtung nach Anspruch 3 oder 4, dadurch gekennzeichnet, daß das Steuerventil ( 20 ) einen Anschlußstutzen ( 4 ) für die Steuerleitung ( 3 ) und Einlaßöffnungen ( 37 ) für das gasförmige Steuermedium aufweist, wobei zwischen den Einlaßöffnungen und dem Anschlußstutzen ( 4 ) ein mittels einer Außenhülse ( 42 ) betätigbares Nadelventil ( 36 ) vorgesehen ist.

6. Vorrichtung nach Anspruch 5, dadurch gekennzeichnet, daß die Außenhülse ( 42 ) mittels einer Rasteinrichtung ( 43 ) in verschiedene, unterschiedlichen Hülsenöffnungen des Nadelventils ( 36 ) zugeordneten Raststellungen einstellbar ist.

7. Vorrichtungen nach einem der vorangegangenen Ansprüche, dadurch gekennzeichnet, daß am Auslaß des Spülrohres ( 34 ) innerhalb des medizinischen Instrumentes ( 5 ) ein Nadelventil ( 44 ) angeordnet ist, dessen Ventilnadel ( 45 ) zur Ausbildung unterschiedlicher Ventilöffnungen einstellbar ist.

8. Vorrichtung nach einem der vorangegangenen Ansprüche dadurch gekennzeichnet, daß die Druck-und die Saugpumpe ( 13,14 ) aus zwei Peristaltik-/ Rollenrad-Pumpen ( 25, 26 ) gebildet sind, die mittels eines Umschaltgetriebes ( 24 ) abwechselnd zum Spülen und Saugen einstellbar und von einem gemeinsamen Antriebsmotor ( 21 ) angetrieben sind.

9. Vorrichtung nach Anspruch 8, dadurch gekennzeichnet, daß die beiden Rollenräder ( 25, 26 ) der Peristaltik-Pumpen( 13,14 ) auf koaxial ineinander angeordneten Achsen ( 27,28 ) des Umschaltgetriebes ( 24 ) gelagert sind.

10. Vorrichtung nach Anspruch 9, dadurch gekennzeichnet, daß beide Rollenradpumpen ( 13,14 ) einen gemeinsamen Anpreßbügel haben.

11. Vorrichtung nach einem der vorangegangenen Ansprüche dadurch gekennzeichnet, daß für die zum Spülen und Absaugen dienenden Schlauchleitungen ( 48,49 ) Halteelemente ( 46,47 ) vorgesehen sind, die beide Schlauchleitungen ( 48,49 ) innerhalb der Rollenradpumpen ( 13,14 ) formschlüssig zueinander lagern und deren Tüllen unterschiedliche Durchmesser aufweisen, so daß eine Vertauschung der Schlauchleitungen nicht möglich ist .

FIG.1

FIG.2

FIG.3

FIG.4

EP 0 423 062 A2

FIG.5

FIG.6

8